# EUROPEAN PATENT APPLICATION

(11) **EP 4 678 630 A1**
(43) Date of publication of application: **14.01.2026**
(21) Application number: 24767300.7
(22) Date of filing: 08.02.2024
(51) Int. Cl.: C07C 253/30, C07C 255/04, C07C 255/02, C07C 255/19

(54) **METHOD FOR PREPARING ALIPHATIC NITRILE-BASED COMPOUND**

(30) Priority: 03.03.2023 KR 20230028352; 13.07.2023 KR 20230090939
(71) Applicant: Korea Kumho Petrochemical Co., Ltd., Seoul 04542 (KR)
(72) Inventor: ROH, Kee Yoon, Daejeon 34021 (KR); CHO, Nam Hyun, Daejeon 34304 (KR); KIM, Hee Su, Sejong 30124 (KR); JANG, Sang Hun, Asan-si, Chungcheongnam-do 31530 (KR); CHOI, Jung Hei, Daejeon 34048 (KR)
(74) Representative: Vossius & Partner Patentanwälte Rechtsanwälte mbB
(86) International application number: PCT/KR2024/001905
(87) International publication number: WO 2024/186011

(57) **Abstract**

The present disclosure relates to a method for preparing an aliphatic nitrile-based compound by directly substituting an aliphatic compound having two or more carboxyl groups with a nitrile-based compound.

## Description

### [Technical Field]

The present disclosure relates to a method for preparing an aliphatic nitrile-based compound, and more particularly, to an environmentally friendly method for preparing an aliphatic nitrile-based compound.

### [Background Art]

With the recent rapid development of information and communication technologies, the market for portable electronic devices such as wireless mobile phones, notebook computers, and tablet computers has been growing. As a result, the demand for secondary batteries used as power sources for these electronic devices has also increased. This increase in demand has led to the advancement of secondary battery technologies and the expansion of their application to electric vehicles and energy storage systems.

The usage time of portable electronic devices, the driving range of electric vehicles, and the capacity of energy storage systems are primarily determined by the performance of the secondary batteries. Lithium secondary batteries have been the focus of most research and development due to their higher discharge voltage and energy density compared to conventional alkaline secondary batteries. In particular, there has been increasing demand for lithium secondary batteries with high power, large capacity, and long lifespan.

A lithium secondary battery is manufactured by injecting an organic electrolyte into a battery cell comprising a cathode that includes a cathode active material capable of intercalating and deintercalating lithium, an anode that includes an anode active material capable of intercalating and deintercalating lithium, and a separator that insulates the cathode and the anode. Copper used as a current collector for the anode is easily oxidized during overdischarge of the secondary battery. As a result, there is a problem in that the lifespan and capacity of the lithium secondary battery are reduced.

To address this issue, aliphatic nitrile-based compounds such as adiponitrile are used as additives in the electrolyte. These aliphatic nitrile-based compounds can suppress the oxidation of copper without interfering with the formation of a solid electrolyte interface (SEI) layer or impairing other characteristics of the secondary battery.

Conventionally, aliphatic nitrile-based compounds have been prepared by chlorinating olefins followed by reaction with cyanide, or by hydrocyanating olefins in the presence of a nickel catalyst. These methods are economically disadvantageous due to the use of catalysts, or result in the generation of impurities, making it difficult to obtain high-purity products even after purification processes. In addition, when aliphatic nitrile-based compounds are prepared in organic solvents to suppress impurity generation, wastewater is produced during washing processes, posing environmental concerns.

Accordingly, there is a growing demand for a process for preparing high-purity aliphatic nitrile-based compounds in an economical and environmentally friendly manner.

### [Disclosure]

### [Technical Problem]

The present disclosure has been devised in consideration of the problems of the conventional technologies described above, and an object thereof is to provide a high-purity aliphatic nitrile-based compound by means of an environmentally friendly method that does not require a washing process, which was essential in conventional preparation methods due to the use of catalysts or other organic reactions.

In addition, according to the present disclosure, high-purity aliphatic nitrile-based compounds can be prepared by a simple method, while enabling easy recycling of the reactants.

### [Technical Solution]

According to an aspect, a method for preparing an aliphatic nitrile-based compound is provided, the method comprising: (a) preparing a mixture comprising a nitrile-based compound and a carbonyl-based compound; and (b) reacting the mixture, wherein the carbonyl-based compound is an aliphatic compound comprising two or more carboxyl groups, and the reaction of step (b) is a reaction in which each of the two or more carboxyl groups is directly substituted with a nitrile group.

In an embodiment, the nitrile-based compound may be one or more selected from the group consisting of hydrogen cyanide, acetonitrile, acrylonitrile, butyronitrile, isobutyronitrile, pivalonitrile, succinonitrile, fumaronitrile, crotononitrile, and benzonitrile.

In an embodiment, the carbonyl-based compound may be at least one selected from the group consisting of adipic acid, pimelic acid, suberic acid, azelaic acid, sebacic acid, undecanedioic acid, and dodecanedioic acid.

In an embodiment, the mixture of step (a) may consist of the nitrile-based compound and the carbonyl-based compound.

In an embodiment, the content of the nitrile-based compound in step (a) may be 1 to 500 parts by weight based on 1 part by weight of the carbonyl-based compound.

In an embodiment, the water content of the mixture in step (a) may be 6,000 ppm or less.

In an embodiment, the reaction of step (b) may be carried out under conditions of 260 to 500°C and 40 to 200 bar.

In an embodiment, step (b) may be performed for 1 to 500 minutes.

In an embodiment, the method may further comprise, after step (b): (c) separating the product obatined in step (b) to obtain the aliphatic nitrile-based compound.

In an embodiment, the purity of the obtained aliphatic nitrile-based compound may be 60 wt% or more.

### [Advantageous Effects]

According to an aspect, an aliphatic nitrile-based compound can be prepared by a simple method.

According to another aspect, the aliphatic nitrile-based compound can be prepared in an environmentally friendly manner by enabling reuse of raw materials and recovery of the product without a separate washing process.

According to yet another aspect, a high-purity aliphatic nitrile-based compound can be prepared.

The effects of an aspect of the present disclosure are not limited to the effects described above, and should be understood to include all effects that can be inferred from the detailed description or the configurations set forth in the claims of the present disclosure.

### [Modes of the Invention]

Hereinafter, an embodiment of the present disclosure will be described. However, the present disclosure may be implemented in various forms and is not limited to the embodiments described herein.

Throughout the specification, when a part is described as being "connected" to another part, it includes not only cases where they are "directly connected" but also cases where they are "indirectly connected" with another element interposed therebetween. In addition, when a part is said to "include" a component, this means that other components may be further included, not excluded, unless specifically stated otherwise.

When a numerical range is described in the present disclosure, unless a more specific range is provided, the value should be interpreted according to the standard rules for significant figures in chemistry. For example, the value "10" encompasses the range from 5.0 to 14.9, and the value "10.0" encompasses the range from 9.50 to 10.49. Furthermore, when multiple numerical values are listed, unless expressly stated otherwise, such examples include all intermediate values. For instance, "x₁, x₂, x₃, or x₄" includes ranges such as "x₁ to x₂," "x₁ to x₃," "x₁ to x₄," "x₂ to x₃," "x₂ to x₄," or "x₃ to x₄."

In the present disclosure, the term "supercritical condition" refers to a state that exceeds the critical point, which is the end point of a phase equilibrium curve. For example, if the critical temperature and critical pressure of compound A are T_{c} and P_{c}, respectively, then the supercritical condition of compound A refers to a state in which the temperature is equal to or higher than T_{c} and the pressure is equal to or higher than P_{c}.

Hereinafter, an embodiment of the present disclosure will be described in detail.

### Method for Preparing an Aliphatic Nitrile-Based Compound

A method for preparing an aliphatic nitrile-based compound according to an embodiment may comprise: (a) preparing a mixture comprising a nitrile-based compound and a carbonyl-based compound; and (b) reacting the mixture, wherein the carbonyl-based compound is an aliphatic compound comprising two or more carboxyl groups, and the reaction in step (b) is a reaction in which each of the two or more carboxyl groups is directly substituted with a nitrile group.

The nitrile-based compound may be at least one selected from the group consisting of hydrogen cyanide, acetonitrile, acrylonitrile, butyronitrile, isobutyronitrile, pivalonitrile, succinonitrile, fumaronitrile, crotononitrile, and benzonitrile, but is not limited thereto.

The carbonyl-based compound may be a compound having two or more carboxyl groups. In addition, the carbonyl-based compound may have at least one carboxyl group at a terminal of the main chain. For example, the carbonyl-based compound may have one or more carboxyl groups at each of both terminals of the main chain, or may have one or more carboxyl groups at one terminal of the main chain and one or more carboxyl groups on a side chain, or may have one or more carboxyl groups at each of both terminals of the main chain and one or more carboxyl groups on a side chain.

In an example, the carbonyl-based compound may be a compound represented by Chemical Formula 1, but is not limited thereto.

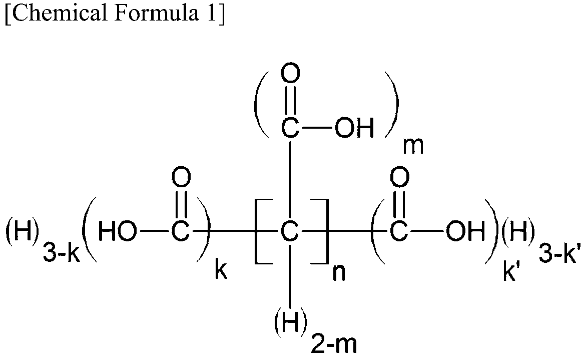

In the above chemical formula, n is an integer of 3 or more, m is an integer from 0 to 2 determined for each of the n carbon chains, k and k' are each an integer from 0 to 3, and k + k' + m may be 2 or more.

In the above chemical formula, n is an integer of 3 or more and, for example, may be 3, 4, 5, 6, 7, 8, 9, 10, 11, 12, 13, 14, or 15, but is not limited thereto. If the value of n falls outside the above range, only a portion of the carboxyl groups of the compound may be substituted with nitrile groups, or it may become difficult to mix the nitrile-based compound and the carbonyl-based compound.

In the above chemical formula, m represents the number of carboxyl groups on side chains and may be an integer from 0 to 2 for each of the n carbons constituting the main chain. In an example, m may be 0 or 1 for each carbon.

In the above chemical formula, k and k' represent the number of carboxyl groups at the ends of the main chain and may each be an integer from 0 to 3. For example, when k or k' is 1, it means that one carboxyl group is present at the terminal of the main chain. In an example, k and k' may each be 0 or 1, satisfying the condition k + k' > 0. When the density of carboxyl groups in a certain region of the carbonyl-based compound is excessively high, only a portion of the carboxyl groups may be substituted with nitrile groups.

For example, the carbonyl-based compound may be at least one selected from the group consisting of adipic acid, pimelic acid, suberic acid, azelaic acid, sebacic acid, undecanedioic acid, and dodecanedioic acid, but is not limited thereto.

In an example, the carbonyl-based compound may have a total number of carbon-carbon bonds associated with the carbons located between each carboxyl group of 7 or more, or 8 or more. For example, adipic acid includes four alkylene carbons between its two carboxyl groups. Since each alkylene carbon has two carbon-carbon bonds and two carbon-hydrogen bonds, the total number of carbon-carbon bonds associated with the carbons located between the carboxyl groups in adipic acid is 8. Although the present disclosure is not limited to such a range, when a carbonyl-based compound satisfying the above condition is used in the nitrile substitution reaction, the resulting product in which all carboxyl groups are substituted with nitrile groups may exhibit excellent purity and yield. In contrast, if the carbonyl-based compound does not satisfy the above condition, only a portion of the carboxyl groups may be substituted with nitrile groups, or a compound different from the intended one may be produced.

In step (b), the reaction of the mixture may be performed without any separate additive such as ammonia, highly concentrated oxygen, or a catalyst. Since the reaction in step (b) may proceed without a separate additive, the mixture in step (a) may consist of the nitrile-based compound and the carbonyl-based compound, but is not limited thereto. For example, the mixture may be prepared by dissolving the carbonyl-based compound in a nitrile-based compound solvent, but is not limited thereto.

In step (a), the content of the nitrile-based compound may be 1 to 500 parts by weight, based on 1 part by weight of the carbonyl-based compound. For example, the content of the nitrile-based compound may be 1, 5, 10, 15, 20, 25, 30, 35, 40, 45, 50, 55, 60, 65, 70, 75, 80, 85, 90, 95, 100, 105, 110, 115, 120, 125, 130, 135, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, or 500 parts by weight, or a range between any two of these values, based on 1 part by weight of the carbonyl-based compound. The higher the content of the nitrile-based compound relative to the carbonyl-based compound, the higher the purity of the resulting product may be. However, if the amount of the nitrile-based compound is excessively high, it may be disadvantageous in terms of economic feasibility.

In step (a), the water content of the mixture may be 6,000 ppm or less. For example, the water content of the mixture may be less than 6,000 ppm, less than 5,000 ppm, less than 4,000 ppm, less than 3,000 ppm, less than 2,000 ppm, less than 1,000 ppm, less than 750 ppm, less than 500 ppm, or less than 250 ppm. As the water content of the mixture decreases, the purity of the product may increase.

The reaction in step (b) may proceed when the temperature and pressure conditions exceed the critical point of the nitrile-based compound. Step (b) may be performed under conditions of 260 to 500°C and 40 to 200 bar. For example, step (b) may be carried out at a reaction temperature of 260°C, 265°C, 270°C, 275°C, 280°C, 285°C, 290°C, 295°C, 300°C, 305°C, 310°C, 315°C, 320°C, 325°C, 330°C, 335°C, 340°C, 345°C, 350°C, 355°C, 360°C, 365°C, 370°C, 375°C, 380°C, 385°C, 390°C, 395°C, 400°C, 405°C, 410°C, 415°C, 420°C, 425°C, 430°C, 435°C, 440°C, 445°C, 450°C, 455°C, 460°C, 465°C, 470°C, 475°C, 480°C, 485°C, 490°C, 495°C, 500°C, or within a range between any two of these values. For example, step (b) may be performed at a reaction pressure of 40 bar, 45 bar, 50 bar, 55 bar, 60 bar, 65 bar, 70 bar, 75 bar, 80 bar, 85 bar, 90 bar, 95 bar, 100 bar, 105 bar, 110 bar, 115 bar, 120 bar, 125 bar, 130 bar, 135 bar, 140 bar, 145 bar, 150 bar, 155 bar, 160 bar, 165 bar, 170 bar, 175 bar, 180 bar, 185 bar, 190 bar, 195 bar, 200 bar, or within a range between any two of these values. If the reaction temperature in step (b) is excessively low, the purity of the product may decrease or the reaction may not proceed. If the reaction temperature is excessively high, the generation of by-products may increase, thereby lowering the purity. If the reaction pressure in step (b) is too low, the reaction may not proceed, and if the pressure is too high, safety issues may arise.

In an example, when the nitrile-based compound is hydrogen cyanide, step (b) may be carried out at 183.5°C or higher and 50 bar or higher. When the nitrile-based compound is acetonitrile, step (b) may be carried out at 272°C or higher and 48.7 bar or higher. When the nitrile-based compound is acrylonitrile, step (b) may be carried out at 267°C or higher and 46 bar or higher. When the nitrile-based compound is butyronitrile, step (b) may be carried out at 309°C or higher and 37.8 bar or higher. When the nitrile-based compound is isobutyronitrile, step (b) may be carried out at 336°C or higher and 40 bar or higher. When the nitrile-based compound is pivalonitrile, step (b) may be carried out at 343°C or higher and 34.4 bar or higher. In other cases, the conditions of step (b) may vary depending on the type of nitrile-based compound used. Accordingly, the above conditions are merely exemplary and do not limit the scope of the present disclosure. The nitrile-based compound may serve both as a solvent and as a reactant.

Step (b) may be performed for 1 to 500 minutes. For example, step (b) may be performed for 1, 5, 10, 15, 20, 25, 30, 35, 40, 45, 50, 55, 60, 65, 70, 75, 80, 85, 90, 95, 100, 105, 110, 115, 120, 125, 130, 135, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, 460, 465, 470, 475, 480, 485, 490, 495, or 500 minutes, or within a range between any two of these values. As the reaction time of step (b) increases, the purity of the product may improve. However, if the reaction time is excessively long, productivity may decrease.

After step (b), the method may further comprise: (c) separating the product obatined in step (b) to obtain the aliphatic nitrile-based compound. The separation in step (c) may involve separating the produced aliphatic nitrile-based compound from residual compounds and may be performed by various known methods such as distillation. The residual compounds may include, for example, at least one selected from the group consisting of unreacted carbonyl-based compounds, unreacted nitrile-based compounds, and compounds in which only a portion of the carboxyl groups of the carbonyl-based compound have reacted, but are not limited thereto.

The residual compounds separated in step (c) may be reused in step (a). Since the method for preparing the aliphatic nitrile-based compound can be performed without the use of separate additives such as catalysts, the residual compounds may be reused without separate purification.

The purity of the obtained aliphatic nitrile-based compound may be 60 wt% or more, 65 wt% or more, 70 wt% or more, 75 wt% or more, 80 wt% or more, 85 wt% or more, or 90 wt% or more.

In an example, the aliphatic nitrile-based compound may be represented by the following Chemical Formula 2, but is not limited thereto.

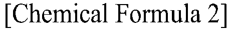

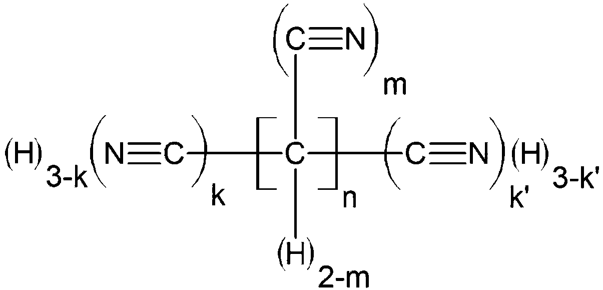

In the above chemical formula, n is an integer of 3 or more, m is an integer from 0 to 2 determined for each of the n carbon chains, and k and k' are each an integer from 0 to 3, wherein k + k' + m may be 2 or more.

The aliphatic nitrile-based compound represented by Chemical Formula 2 may be a compound in which all carboxyl groups of the compound represented by the aforementioned Chemical Formula 1 are substituted with nitrile groups.

The water content of the product obtained by the preparation method may be 500 ppm or less. For example, the water content of the product may be less than 500 ppm, less than 400 ppm, less than 300 ppm, less than 200 ppm, less than 100 ppm, less than 75 ppm, or less than 50 ppm. As the water content of the product decreases, the occurrence of side reactions during storage of the aliphatic nitrile-based compound may be reduced, thereby improving storage stability.

Hereinafter, embodiments of the present disclosure will be described in more detail. However, the experimental results described below are representative examples among the embodiments, and the scope and content of the present disclosure should not be construed as being limited or restricted by the embodiments. The effects of various embodiments of the present disclosure that are not explicitly stated below will be specifically described in the relevant sections.

### Example 1

A reaction system was prepared by introducing 10 parts by weight of adipic acid and 100 parts by weight of acetonitrile into a 1,000 mL reactor (autoclave) equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate acetonitrile and adiponitrile (ADN). The acetonitrile was reused, and the adiponitrile was analyzed using gas chromatography (GC) to determine the purity of the product. In addition, the water content of the obtained product was measured.

### Example 2

A reaction system was prepared by introducing 10 parts by weight of adipic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 350°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate acetonitrile and adiponitrile. The acetonitrile was reused, and the adiponitrile was analyzed using gas chromatography (GC) to determine the purity of the product. In addition, the water content of the obtained product was measured.

### Example 3

A reaction system was prepared by introducing 10 parts by weight of sebacic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate acetonitrile and adiponitrile. The acetonitrile was reused, and the adiponitrile was analyzed using gas chromatography (GC) to determine the purity of the product. In addition, the water content of the obtained product was measured.

### Example 4

A reaction system was prepared by introducing 10 parts by weight of sebacic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 350°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate acetonitrile and adiponitrile. The acetonitrile was reused, and the adiponitrile was analyzed using gas chromatography (GC) to determine the purity of the product. In addition, the water content of the obtained product was measured.

### Example 5

A reaction system was prepared by introducing 10 parts by weight of suberic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate acetonitrile and adiponitrile. The acetonitrile was reused, and the adiponitrile was analyzed using gas chromatography (GC) to determine the purity of the product. In addition, the water content of the obtained product was measured.

### Comparative Example 1

A reaction system was prepared by introducing 10 parts by weight of maleic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate the product and acetonitrile. The acetonitrile was reused, and the product was analyzed using gas chromatography (GC) to determine the purity; however, the target aliphatic nitrile compound was not sufficiently produced.

### Comparative Example 2

A reaction system was prepared by introducing 10 parts by weight of succinic acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate the product and acetonitrile. The acetonitrile was reused, and the product was analyzed using gas chromatography (GC) to determine the purity; however, the target aliphatic nitrile compound was not sufficiently produced.

### Comparative Example 3

A reaction system was prepared by introducing 10 parts by weight of glutaric acid and 100 parts by weight of acetonitrile into a 1,000 mL reactor equipped with a stirrer. The inside of the reactor was purged with nitrogen three times at a pressure of 2-3 bar. While stirring the reactor at 300 rpm under atmospheric pressure, the internal temperature was raised to 280°C. The reaction was carried out at this temperature for 4 hours, and the reaction pressure was 90 bar. After completion of the reaction, the reaction system was cooled to room temperature. Thereafter, the reaction mixture was subjected to reduced-pressure separation to separate the product and acetonitrile. The acetonitrile was reused, and the product was analyzed using gas chromatography (GC) to determine the purity; however, the target aliphatic nitrile compound was not sufficiently produced.

The reaction conditions and product purities of the Examples and Comparative Examples are summarized in Table 1 below.

**[Table 1]**

| Classification | S1 | S2 | Stirring Speed (rpm) | Reactio n Temper ature (° C) | Reactio n Pressure (bar) | Reactio n Time (hr) | Water Content (ppm) | Purity (%) |
|---|---|---|---|---|---|---|---|---|
| Example 1 | Adpic acid | Acetoni trile | 300 | 280 | 90 | 4 | 26 | 85.8 |
| Example 2 | Adipic acid | Acetoni trile | 300 | 350 | 90 | 4 | 25 | 95.2 |
| Example 3 | Sebacic acid | Acetoni trile | 300 | 280 | 90 | 4 | 29 | 95.1 |
| Example 4 | Sebacic acid | Acetoni trile | 300 | 350 | 90 | 4 | 31 | 96.5 |
| Example 5 | Sebacic acid | Acetoni trile | 300 | 280 | 90 | 4 | 28 | 96.5 |
| Comparative Example 1 | Maleic acid | Acetoni trile | 300 | 280 | 90 | 4 | - | - |
| Comparative Example 2 | Succini c acid | Acetoni trile | 300 | 280 | 90 | 4 | - | - |
| Comparative Example 3 | Glutaric acid | Acetoni trile | 300 | 280 | 90 | 4 | - | - |
| -S1: Aliphatic acid compound | | | | | | | | |
| -S2: Nitrile-based compound | | | | | | | | |

Referring to the table above, among the aliphatic acid compounds having two or more carboxylic acids, compounds such as maleic acid, succinic acid, and glutaric acid were found to produce little or no aliphatic nitrile-based compounds when reacted with a nitrile-based compound, whereas compounds such as adipic acid, sebacic acid, and suberic acid successfully produced aliphatic nitrile-based compounds with high purity.

The aforementioned description of the present disclosure is provided by way of example and those skilled in the art will understand that the present disclosure can be easily changed or modified into other specified forms without change or modification of the technical spirit or essential characteristics of the present disclosure. Therefore, it should be understood that the aforementioned examples are only provided by way of example and not provided to limit the present disclosure. For example, each of constituents described as a single form may be separately implemented and, similarly, constituents described as being separated may be implemented in a combined form.

It should be understood that the scope of the present disclosure is defined by the following claims and the invention is to cover all modifications, equivalents, and alternatives falling within the spirit and scope of the invention as defined by the claims.

## Claims

1. A method for preparing an aliphatic nitrile-based compound, comprising:
(a) preparing a mixture comprising a nitrile-based compound and a carbonyl-based compound; and
(b) reacting the mixture,
wherein the carbonyl-based compound is an aliphatic compound comprising two or more carboxyl groups, and
wherein the reaction of step (b) is a reaction in which each of the two or more carboxyl groups is directly substituted with a nitrile group.

2. The method for preparing an aliphatic nitrile-based compound according to claim 1, wherein the nitrile-based compound is one or more selected from the group consisting of hydrogen cyanide, acetonitrile, acrylonitrile, butyronitrile, isobutyronitrile, pivalonitrile, succinonitrile, fumaronitrile, crotononitrile, and benzonitrile.

3. The method for preparing an aliphatic nitrile-based compound according to any one of claims 1 and 2, wherein the carbonyl-based compound is at least one selected from the group consisting of adipic acid, pimelic acid, suberic acid, azelaic acid, sebacic acid, undecanedioic acid, and dodecanedioic acid.

4. The method for preparing an aliphatic nitrile-based compound according to any one of claims 1 to 3, wherein the mixture of step (a) consists of the nitrile-based compound and the carbonyl-based compound.

5. The method for preparing an aliphatic nitrile-based compound according to any one of claims 1 to 4, wherein in step (a), the content of the nitrile-based compound is 1 to 500 parts by weight based on 1 part by weight of the carbonyl-based compound.

6. The method for preparing an aliphatic nitrile-based compound according to any one of claims 1 to 5, wherein in step (a), the water content of the mixture is 6,000 ppm or less.

7. The method for preparing an aliphatic nitrile-based compound according to any one of claims 1 to 6, wherein the reaction of step (b) is performed under conditions of 260 to 500°C and 40 to 200 bar.

8. The method for preparing an aliphatic nitrile-based compound according to any one of claims 1 to 7, wherein step (b) is performed for 1 to 500 minutes.

9. The method for preparing an aliphatic nitrile-based compound according to any one of claims 1 to 8, further comprising, after the step (b):
(c) separating the product of step (b) to obtain the aliphatic nitrile-based compound.

10. The method for preparing an aliphatic nitrile-based compound according to claim 9, wherein the purity of the obtained aliphatic nitrile-based compound is 60 wt% or more.
